Europäisches Patentamt

European Patent Office

Office européen des brevets

(11) Veröffentlichungsnummer: **0 090 327**
**B1**

(12) EUROPÄISCHE PATENTSCHRIFT

(45) Veröffentlichungstag der Patentschrift: **20.01.88**

(21) Anmeldenummer: **83102824.6**

(22) Anmeldetag: **22.03.83**

(51) Int. Cl.⁴: **A 61 B 10/00**

(54) Vorrichtung zur Bestimmung des Eisprungs bei Frauen.

(30) Priorität: **29.03.82 DE 3211573**
**09.10.82 DE 3237565**

(43) Veröffentlichungstag der Anmeldung:
**05.10.83 Patentblatt 83/40**

(45) Bekanntmachung des Hinweises auf die
Patenterteilung:
**20.01.88 Patentblatt 88/03**

(84) Benannte Vertragsstaaten:
**AT BE CH DE FR GB IT LI LU NL SE**

(56) Entgegenhaltungen:
**EP-A-0 031 251**
**DD-A- 147 046**
**DE-A-1 766 548**
**US-A-4 148 304**
**US-A-4 151 831**

**Die Akte enthält technische Angaben, die nach dem Eingang der Anmeldung eingereicht wurden und die nicht in dieser Patentschrift enthalten sind.**

(73) Patentinhaber: **Weiland, Werner**
**Koblenz-Olper-Strasse 172**
**D-5413 Bendorf-Sayn (DE)**

(72) Erfinder: **Weiland, Werner**
**Koblenz-Olper-Strasse 172**
**D-5413 Bendorf-Sayn (DE)**

(74) Vertreter: **Quermann, Helmut, Dipl.-Ing.**
**Postfach 6145 Gustav-Freytag-Strasse 25**
**D-6200 Wiesbaden (DE)**

## Beschreibung

Die Erfindung betrifft eine Vorrichtung zur Bestimmung des Eisprunges bei Frauen, die einen mittels eines Bandes an einer Extremität des Körpers befestigbaren, auf die Haut auflegbaren Temperaturfühler, einen mit dem Temperaturfühler verbundenen Mikroprozessor sowie eine mit dem Mikroprozessor verbundene, gleichfalls mittels eines Bandes mit einer Extremität des Körpers befestigbare Anzeige aufweist.

Zur Verhütung der Empfängnis ist es u.a. bekannt, daß die Frau ihre Körpertemperatur an solchen Tagen mißt, an denen sie mit dem Eisprung rechnet. Erhöht sich ihre Körpertemperatur um einen bestimmten Wert gegenüber ihrer Normaltemperatur, so ist dies ein Zeichen für das Vorliegen des erfolgten Eisprunges. Die genannte Temperaturmeßmethode dient gleichzeitig der Aussage über den empfängnisgünstigen Zeitpunkt. Die Kenntnis dieses Zeitpunkts ist dann erwünscht, wenn eine gezielte Befruchtung der reifen Eizelle erfolgen soll. Die Befruchtung der Eizelle ist vorzunehmen, wenn eine gegenüber der Normaltemperatur erhöhte Körpertemperatur gemessen wird, die den Eisprung signalisiert. Diese Temperaturerhöhung ist bei einer Frau üblicherweise gleich. Zeitlich veränderlich ist jedoch bei ein und der selben Frau die entsprechende Normaltemperatur, die innerhalb gewisser Grenzen schwanken kann, wobei diese Schwankungen beispielsweise durch Veränderungen des Stoffwechsels, Veränderungen der den Körper umgebenden Temperatur sowie durch Veränderungen des Gesundheitszustandes verursacht sein können. Es ist zudem denkbar, daß sich eine veränderte Normaltemperatur aufgrund unterschiedlicher psychologischer Situationen, in denen sich eine Frau befindet, einstellt. Eine Bestimmung des Eisprungs durch die genannte Temperaturmeßmethode ist für eine Frau somit aufwendig, weil es für sie nicht nur möglich ist, täglich ihre Körpertemperatur zu messen, sondern ebenfalls zur Bestimmung der Eisprungtemperaturdifferenz ihre Normaltemperatur zu kennen.

Aus der DD—A—147 046 ist eine Vorrichtung zur Bestimmung des Eisprungs bei Frauen gemäß dem Oberbegriff bekannt, die in Art einer Uhr ausgebildet ist und mittels eines Bandes im Bereich des Handgelenks der Frau befestigt werden kann. Im befestigten Zustand liegt der Temperaturfühler auf der Haut auf, mit diesem ist der Mikroprozessor und die Anzeige verbunden. Der Temperaturfühler ist als Thermistor ausgebildet, er mißt die Körpertemperatur und führt die Meßwerte dem Mikroprozessor zu. Mit der Vorrichtung wird die Körpertemperatur gemessen, die Messung erfolgt, wenn die Vorrichtung getragen wird, täglich zu einem bestimmten Zeitpunkt, kontinuierlich oder in kurzen Intervallen, in den beiden letztgenannten Fällen wird aus den Daten der Tag des Anstiegs der Körpertemperatur durch Bildung von Durchschnittswerten oder nach speziellen Programmen ermittelt, so daß die Vorrichtung nicht ständig getragen werden muß. Alle Teile der Vorrichtung sind in einem gemeinsamen Gehäuse untergebracht.

Aus der US—A—4 151 831 ist ferner eine in Art einer Uhr ausgebildete Vorrichtung der genannten Art bekannt, bei der der Temperaturfühler jedoch in einen am Uhrengehäuse schwenkbar angelenkten Ring integriert ist. Mit der Vorrichtung kann die Frau ihre Körpertemperatur bestimmen, indem sie den Ring vom Gehäuse wegklappt und das den Temperaturfühler aufnehmende Ringteil in den Mund einführt, wobei über die sonst als Zeitanzeige dienende Anzeige der Temperaturwert ausgewiesen wird.

Es ist Aufgabe vorliegender Erfindung, eine Vorrichtung der eingangs genannten Art so zu gestalten, daß eine große Sicherheit bei der Bestimmung des Eisprunges erreicht wird, bei gleichzeitiger einfacher sicherer Handhabung.

Die Aufgabe wird bei einer Vorrichtung der eingangs genannten Art dadurch gelöst, daß der Temperaturfühler auf der Innenseite des Oberarms angeordnet werden kann und mittels einer Leitung mit der Anzeige verbunden ist, die am Handgelenk angeordnet werden kann, wobei der Temperaturfühler während des Schlafs die momentanen Temperaturen ermittelt und den jeweiligen Temperaturwert über die Leitung einem Signalgeber mit in mehreren Stromkreisen vorgesehenen variablen Widerständen zu Abstimmzwecken mit einem NTC-Widerstand zuführt, in den Mikroprozessor ein Basiswert, der der Normaltemperatur der Frau entspricht, und ein Differenzwert, der der Eisprungtemperaturdifferenz der Frau entspricht, eingegeben sind und die mit dem Mikroprozessor verbundene Anzeige bei einer bestimmten Temperaturdifferenz den Signalgeber betätigt.

Erfindungsgemäß ist damit vorgesehen, mit dem Temperaturfühler Temperaturdifferenzen zu ermitteln, da ausschließlich diese für die Bestimmung des Eisprungs aussagekräftig sind. Der Einsatz der Vorrichtung erfolgt derart, daß dann, wenn die Körpertemperatur der Frau noch deren Normaltemperatur entspricht, die Vorrichtung angelegt und über den Temperaturfühler die Normaltemperatur der Frau gemessen wird. Der Normaltemperatur wird ein Basiswert zugeordnet, der in den Mikroprozessor eingegeben und gespeichert wird. Von hier ist er jederzeit abrufbar und auf der Anzeige optisch darstellbar. Zu dem Basiswert läßt sich ein Differenzwert bzw. Referenzwert in den Mikroprozessor eingeben, der in Bezug auf den Basiswert einer solchen Erhöhung der Körpertemperatur der Frau entspricht, die Rückschlüsse auf den Eisprung zuläßt. Auch dieser Differenzwert ist jederzeit vom Mikroprozessor abrufbar und über die Anzeige optisch darstellbar. Es ist also nicht nötig, daß die Frau ihre Normaltemperatur kennt, von Interesse sind ausschließlich der der Normaltemperatur zugeordnete Basiswert und der Differenzwert.

Der Temperaturfühler ist derart aufgebaut, daß die Temperaturdifferenzen durch einen temperaturabhängigen Widerstand bestimmt werden. Die Ermittlung des Basiswertes, der in Zusammenhang mit der Normaltemperatur steht, erfolgt durch die Abgleichung einer entsprechenden Schaltung im Signalgeber. Der Signalgeber besteht vorteilhaft aus einem Wählteil mit dazugehöriger Skala, dem eigentlichen Signalgeber, der als akustischer Signalgeber oder als mit ihrer Oberfläche dem Körper zugewandte Wärmeplatte ausgebildet ist, einem Dreipositionsschalter sowie einer Batterie als Energieträger für die Vorrichtung. Der Basiswert kann dadurch ermittelt werden, daß mehrere Stromkreise mit variablen Widerständen solange durchgeschaltet werden, bis ein Widerstand eines Stromkreises exakt auf einen eine definierte Temperatur repräsentierenden NTC-Widerstand abgestimmt ist. Das Durchschalten als solches erfolgt über das drehbare Wählteil, wobei aus Zweckmäßigkeitsgründen der Dreipositionsschalter derart eingestellt ist, daß im Fall der Abstimmung der beiden Widerstände der im Signalgeber befindliche akustische Signalgeber betätigt wird. Es ist jedoch gleichfalls möglich, den Dreipositionsschalter so zu schalten, daß nur die Wärmeplatte betätigt wird oder daß sowohl Wärmeplatte als auch akustischer Signalgeber zusammen in Betrieb gesetzt werden. Für die Bestimmung des Basiswertes ist demnach das Kennen der Normaltemperatur überflüssig, der Einfachheit halber sollte dem Basiswert der Wert Null zugewiesen werden. Da die gegenüber der Normaltemperatur erhöhte Temperatur, bei der der Eisprung stattfindet, einen Erfahrungswert darstellt, wird entsprechend dieser Temperaturdifferenz das Wählteil auf der Skala um Temperaturdifferenzen repräsentierende Einheiten in Richtung steigender Werte verdreht. Dies hat zur Folge, daß das Signal des akustischen Signalgebers unterbrochen wird. Hat der Eisprung stattgefunden, wird der im Signalgeber befindliche akustische Signalgeber wieder betätigt und somit die Information an die die Vorrichtung tragende Frau weiter gegeben. Durch wahlweise Schaltung des Dreipositionenschalters kann es jedoch auch möglich sein, daß der Signalgeber eine rein thermische bzw. eine kombinierte akustisch-thermische Information über das Vorliegen des Eisprunges abgibt.

Vorteilhaft ist im Gehäuse gleichzeitig eine Uhr angeordnet. Über eine Steuerleitung zwischen der Uhr und dem Mikroprozessor ist es dann möglich, Zeitpunkte, an denen eine Temperaturermittlung stattfinden soll, vorab einzustellen. Einer Frau ist es dann möglich, nachts, wenn sie schläft, die Temperatur zu ermitteln und aufgrund der Speichermöglichkeiten des Mikroprozessors diese Temperatur morgens nach dem Aufstehen aus dem Mikroprozessor abzurufen und zu lesen. Sie kann damit nachvollziehen, wann ein den Eisprung signalisierender Temperaturanstieg erreicht wurde.

## Patentansprüche

1. Vorrichtung zur Bestimmung des Eisprunges bei Frauen, die einen mittels eines Bandes an einer Extremität des Körpers befestigbaren, auf die Haut auflegbaren Temperaturfühler, einen mit dem Temperaturfühler verbundenen Mikroprozessor sowie eine mit dem Mikroprozessor verbundene, gleichfalls mittels eines Bandes mit einer Extremität des Körpers befestigbare Anzeige aufweist, dadurch gekennzeichnet, daß der Temperaturfühler auf der Innenseite des Oberarms angeordnet werden kann und mittels einer Leitung mit der Anzeige verbunden ist, die am Handgelenk angeordnet werden kann, wobei der Temperaturfühler während des Schlafs die momentanen Temperaturen ermittelt und den jeweiligen Temperaturwert über die Leitung einem Signalgeber mit in mehreren Stromkreisen vorgesehenen variablen Widerständen zu Abstimmzwecken mit einem NTC-Widerstand zuführt, in den Mikroprozessor ein Basiswert, der der Normaltemperatur der Frau entspricht, und ein Differenzwert, der der Eisprungtemperaturdifferenz der Frau entspricht, eingegeben sind und die mit dem Mikroprozessor verbundene Anzeige bei einer bestimmten Temperaturdifferenz den Signalgeber betätigt.

2. Vorrichtung nach Anspruch 1, dadurch gekennzeichnet, daß das am Handgelenk angeordnete Band ein Gehäuse mit Mikroprozessor und Anzeige aufnimmt.

3. Vorrichtung nach Anspruch 1 oder 2, dadurch gekennzeichnet, daß im Gehäuse ein Uhrenteil angeordnet ist.

4. Vorrichtung nach einem der Ansprüche 1 bis 3, gekennzeichnet durch eine zweckmäßig im Gehäuse angeordnete Batterie als Energieträger für die Vorrichtung.

5. Vorrichtung nach einem der Ansprüche 1 bis 4, dadurch gekennzeichnet, daß der Signalgeber als akustischer Signalgeber ausgebildet ist.

6. Vorrichtung nach einem der Ansprüche 1 bis 4, dadurch gekennzeichnet, daß der Signalgeber als Wärmeplatte ausgebildet ist, die mit ihrer Oberfläche dem Körper zugewandt ist.

## Revendications

1. Dispositif pour déterminer l'ovulation chez les femmes, qui comporte un palpeur de température pouvant se fixer au moyen d'une bande à une extrémité du corps et s'appliquant sur la peau, un microprocesseur relié au palpeur de température, ainsi qu'un indicateur relié au microprocesseur et pouvant se fixer également au moyen d'une bande sur une extrémité du corps, dispositif caractérisé en ce que le palpeur de température peut être disposé sur la face intérieure de la partie supérieure du bras et est relié au moyen d'un conduit à l'indicateur, qui peut être disposé sur le poignet, le palpeur de température déterminant pendant le sommeil les températures instantées et envoyant la valeur de température qui se présente, par l'intermédiaire du conduit à un transmetteur de signal avec des résistances variables prévues dans plusieurs circuits dans des buts d'accord avec une résistance NTC, tandis que dans le microprocesseur sont introduites une valeur de base, qui correspond à la température normale de la femme, et une valeur différentielle, qui correspond à la différence de température de l'ovulation de la femme, et l'indicateur relié au microprocesseur actionnant le transmetteur de signal, lors d'une différence de température déterminée.

2. Dispositif selon la revendication 1, caractérisé en ce que la bande disposée sur le poignet sert à loger un boîtier avec microprocesseur et indicateur.

3. Dispositif selon la revendication 1 ou 2, caractérisé en ce qu'un élément d'horlogerie est disposé dans le boîtier.

4. Dispositif selon l'une des revendications 1 à 3, caractérisé par une batterie disposée avantageusement dans le boîtier en tant que porteur d'énergie pour le dispositif.

5. Dispositif selon l'une des revendications 1 à 4, caractérisé en ce que le transmetteur de signal est constitué sous forme de transmetteur de signal acoustique.

6. Dispositif selon l'une des revendications 1 à 4, caractérisé en ce que le transmetteur de signal est constitué sous forme de plaque chauffante, qui est disposée par sa surface en regard du corps.

**Claims**

1. Device for determining ovulation in women, which comprises a temperature sensor which can be secured by means of a strap to an extremity of the body and can be applied to the skin, a microprocessor connected to the temperature sensor and an indicating means which is connected to the microprocessor and can also be secured by means of a strap to an extremity of the body, characterised in that the temperature sensor can be disposed on the inside of the upper arm and is connected by means of a conductor to the indicating means, which can be disposed on the wrist, the temperature sensor determining the momentary temperatures during sleep and feeding the respective temperature value through the conductor to a signal generator comprising variable resistors provided in a number of circuits for purposes of tuning with an NTC resistor, while there are fed into the microprocessor a base value which corresponds to the normal temperature of the woman, and a difference value which corresponds to the ovulation temperature difference of the woman, and the indicating means connected to the microprocessor actuates the signal generator at a particular temperature difference.

2. Device according to claim 1, characterised in that the strap disposed on the wrist accommodates a casing with microprocessor and indicating means.

3. Device according to claim 1 or 2, characterised in that a clock part is disposed in the casing.

4. Device according to one of claims 1 to 3, characterised by a battery appropriately disposed in the casing as an energy carrier for the device.

5. Device according to one of claims 1 to 4, characterised in that the signal generator is in the form of an acoustic signal generator.

6. Device according to one of claims 1 to 4, characterised in that the signal generator is constructed as a hot plate having its surface facing towards the body.